# EUROPEAN PATENT SPECIFICATION

(11) **EP 0 525 317 B1**
(45) Date of publication and mention of the grant of the patent: **19.02.1997**
(21) Application number: 92108412.5
(22) Date of filing: 19.05.1992
(51) Int. Cl.: B01D 63/02

(54) **An apparatus for effecting mass and/or heat transfer**
Vorrichtung zur Bewerkstelligung von Stoff- und/oder Wärmeübertragung
Appareillage pour effectuer le transfert de masse ou de chaleur

(30) Priority: 01.08.1991 SE 9102275
(43) Date of publication of application: 03.02.1993
(73) Proprietor: GAMBRO DIALYSATOREN GMBH & CO. KG, D-72379 Hechingen (DE)
(72) Inventor: Antoni, Roland, W-4403 Senden (DE); Mayer, Georg, W-7450 Hechingen-Boll (DE); Raff, Manfred, Dr., W-7467 Bisingen-Thanheim (DE); Spranger, Kurt, W-7403 Ammerbuch 1 (DE); Volm, Josef, W-7452 Haigerloch-Owingen (DE)
(74) Representative: Asketorp, Göran

(56) References cited:
- EP-A- 0 343 359
- EP-A- 0 360 133
- DD-A- 285 722
- US-A- 4 950 391
- US-A- 4 990 251
- US-A- 5 002 668

## Description

### FIELD OF INVENTION

The present invention relates to an apparatus for effecting mass and/or heat transfer, including a cylindrical open-ended housing closed by two end caps, each provided with an inlet or an outlet for a first fluid intended to flow through a bundle of hollow fibers arranged within the housing between two end walls, at least one of said end caps being provided with an outlet for a second fluid intended to be removed from the space outside the fibers, the inlets and/or outlets provided in one and the same end cap being separated by the adjacent end wall.

Devices of the above kind are used for e.g. different kinds of medical treatments such as hemodialysis, hemofiltration, plasmapheresis and immunotherapy. Other fields of use are, for instance, dialysis in general and filtration in general, for example in connection with cleaning or desalination of sea water. The apparatus according to the invention may also be used as a heat exchanger or as a blood oxygenator.

### PRIOR ART

The apparatus according to the invention is an improvement or a modification of the apparatuses disclosed in, for instance, US-A-4 990 251, US-A-5 002 668, EP-A1-0 305 672 and EP-A-442 310 (published: 21.08.91), which, however, all have the inlets and outlets for the second fluid arranged as nipples on the housing, providing rather complicated tools for the manufacture of the housing.

In US-A-4 724 900, an apparatus is described having said inlets and outlets arranged in the end caps. A disadvantage of said design is that the housing has to be provided with openings in front of the inlets and outlets for one of the fluids.

In WO 88/01895, an apparatus is described having the housing as a cylinder without openings. Moreover, this apparatus has a collar that supports a spigot leading from a fluid inlet. The end cap is composed of several portions requiring elaborate assembly handling operations.

### SUMMARY OF THE INVENTION

The object of the present invention is to provide a very simple and inexpensive housing which can be made either by injection moulding or blow moulding in simple moulds or forms. Also the handling of the apparatus is simplified in connection with the manufacturing of the complete apparatus. In addition, the apparatus is designed in such a way that the end walls, which normally are made of polyurethan, are prevented from reaching the housing.

Accordingly, there is provided an apparatus for effecting mass and/or heat transfer, comprising: a cylindrical housing having first and second open ends interconnected by an inner wall surface; a bundle of hollow fibers, arranged inside the housing and extending between first and second end walls arranged adjacent the first and second open ends, respectively, of the housing; a first end cap having an engagement portion for cooperation with the housing for closing the first open end thereof, comprising first connector means for passing a first fluid intended to flow through the interior of the bundle of hollow fibers, and second connector means for passing a second fluid from an exterior of the bundle of hollow fibers; and a second end cap for closing the second open end of the housing, comprising first connector means for passing the first fluid.

According to the invention, a ring is arranged between the first end wall and the housing adjacent the first open end and the first end cap, for providing a fluid channel for the second fluid from the exterior of the bundle of hollow fibers and between the ring and the inner wall surface to the second connector means.

Preferably, the second end cap is identical with the first end cap. A second ring is arranged between the second end wall and the housing adjacent the second open end and the second end cap, for providing a fluid channel for the second fluid at the exterior of the bundle of hollow fibers and between the ring and the inner wall surface to the second connector means.

Such an apparatus may be used for dialysis and also for pure filtration. However, in connection with filtration, no inlet for the second fluid is necessary.

In order to get a good sealing between the two fluids, which may be liquids and/or gases, the ring is preferably partly moulded into the corresponding end wall.

In a preferred embodiment of the apparatus according to the invention the ring consists of an inner portion abutting the fibers and intended to support the fibers during the moulding of the adjacent end wall and an outer portion abutting the housing and/or the end cap and being supported by the housing and/or the end cap.

Said inner and outer portions are interconnected by several webs forming one or more ducts which are filled with the moulding material in connection with the moulding of the end walls. Thanks to this design, a good sealing is obtained at the same time as the distribution of the moulding material is facilitated.

The mounting of the bundle of hollow fibers is facilitated, if the inner wall surface of the housing and an inner surface of the ring are essentially in line, supporting the bundle of hollow fibers during the moulding of the end walls.

A good sealing is provided, if each end cap is attached to the housing and/or the end wall by glueing. Alternatively, it may, however, also be attached by welding or a screw connection. In connection with those alternatives it may be suitable to include further sealings between the housing, the end caps and the end walls. Examples of such sealings in the form of sealing rings are shown in the above mentioned patents.

A good distribution of the second fluid is provided, if the housing is provided at each end with a widened part providing a peripheral channel for said fluid.

A great advantage of the present invention is that one and the same housing may be combined with different kinds of end caps having different kinds of connection nipples for the connection of tube connectors of different kinds. Further advantages are:

Optimal distribution of dialysate and other fluids around the bundle in the "head area" affected by the thickness, height and number of the fins provided by the house, the end cap and the support ring.

Support of the bundle before and during potting. By variation of the inner diameter of the support ring it is possible to vary the bundle diameter.

The support ring deviates forces caused by e g fiber shrinkage into the housing and reduces therefore the load on the glue seam between the end walls and the headers or end caps.
All connectors may be placed at the headers;
simple injection moulding tool for the housing;
constant wall thickness of the housing allows general minimization of the wall thickness;
the same housing may be used for hemofilters and hemodialyzers;
a symmetric housing is easy to handle during production and assembly.
No direct connection between the end walls and the housing;
recycling of housing possible;
the risk for cracks between the housing and the end walls is eliminated or at least reduced. Such cracks are often created during steam sterilization.

### SHORT DESCRIPTION OF THE DRAWINGS

Fig 1 shows a first embodiment of the apparatus according to the invention.

Fig 2 shows a second embodiment provided with modified end caps.

Fig 3 shows in a larger scale schematically a part of a third embodiment.

### PREFERRED EMBODIMENTS OF THE INVENTION

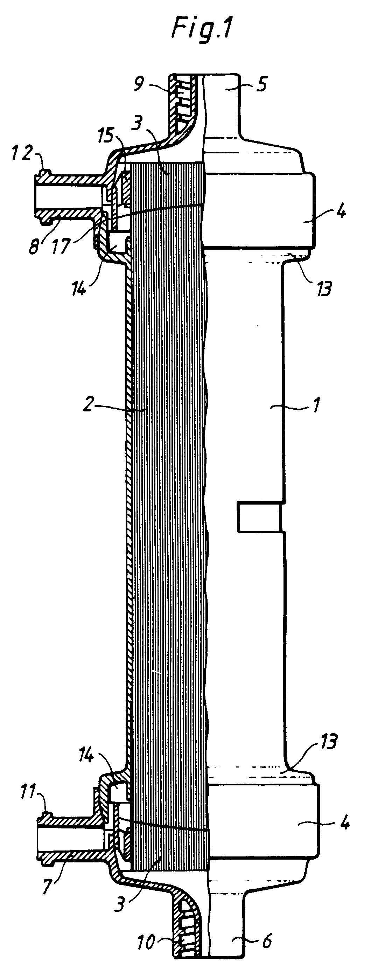

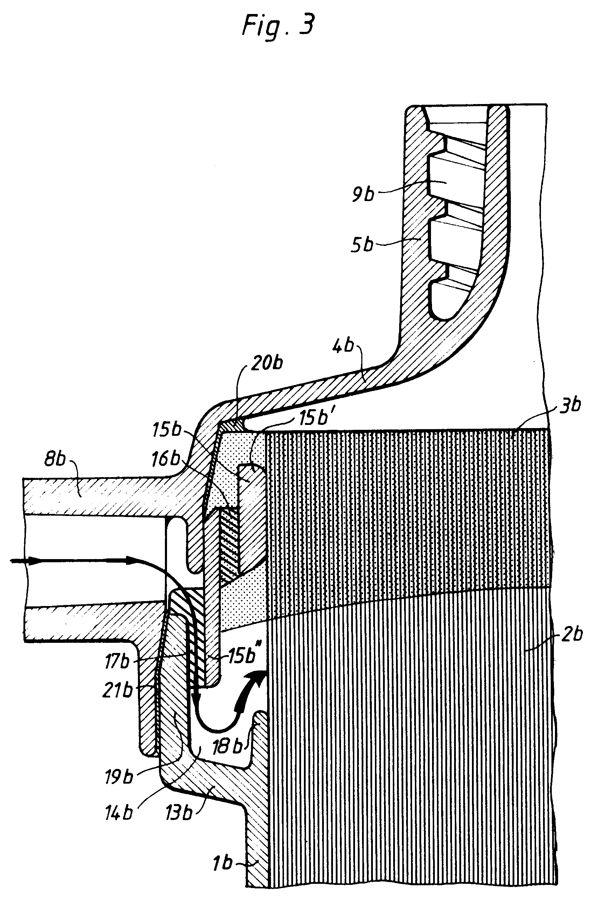

The embodiment shown in fig 1 includes a housing 1 enclosing a bundle of hollow fibers 2 which are arranged between two end walls 3 of moulded polyurethane. The ends of the open ended housing 1 is closed by two end caps 4 with an inlet 5 for a first fluid and an outlet 6 for the same fluid. The end caps 4 are, furthermore, provided with an inlet 7 for a second fluid and an outlet 8 for the same fluid. The inlet 5 and the outlet 6 for the first fluid are provided with an inner screw thread 9 and 10, respectively. The inlet 7 and the outlet 8 for the second fluid are instead provided with a simple external screw connection 11 and 12, respectively. The housing 1 is at its end provided with a widened part 13 providing an inner peripheral channel 14. The polyurethane material of the end walls 3 is prevented from reaching the housing 1 by a ring 15 which is shown in larger scale in fig 3.

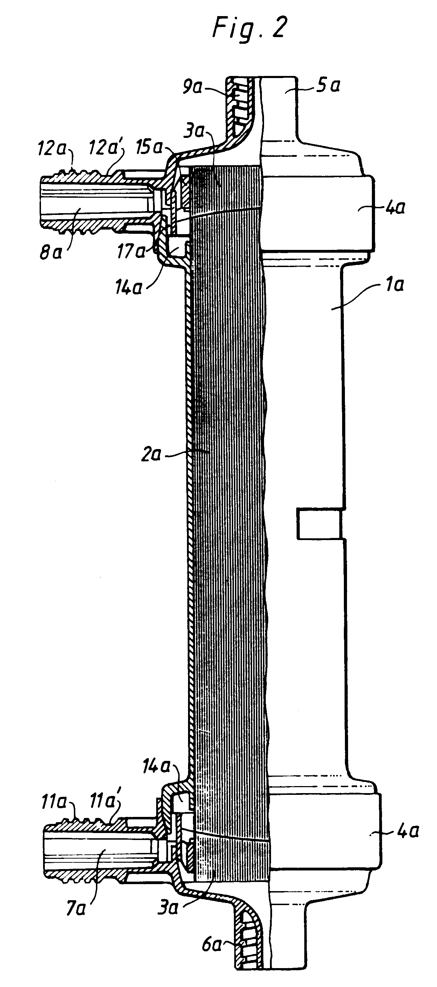

The embodiment shown in fig 2 corresponds essentially to the one shown in fig 1. The same reference numerals have therefore been used for corresponding details, but with the addition of a. The only difference is that the inlet 7a and outlet 8a for the second fluid have been modified by being provided with a more complete outer screw thread 11a and 12a, respectively. Said inlet 7a and said outlet 8a have furthermore been provided with a groove 11a' and 12a', respectively. By the combination of the screw threads 11a and 12a and the grooves 11a' and 12a' the inlet 7a and outlet 8a may be used together with different kinds of tube connectors, as is described more in detail in EP-A-442 310. The screw threads 11a and 12a are intended to be used together with simple screw connectors and the grooves 11a' and 12a' are intended to be used together with more complicated connectors of the so called Hansen-type.

The embodiment shown in fig 3 corresponds essentially to the embodiments shown in figs. 1 and 2. The same reference numerals have therefore been given to corresponding details, but with the addition of b. A bundle of hollow fibers 2b is arranged longitudinally in a housing 1b between two end walls 3b of which only one is shown in the figure. An inlet 5b for a first fluid is provided with an inner screw thread 9b. The nipple 8b is here shown to be an inlet. Consequently, the apparatus according to the invention may be designed either for a concurrent or a counter-current flow. The nipple 8b can also be provided with some kind of tube connecting means. One possibility is, however, that a tube is pressed with a press-fitting upon the nipple 8b. The ring 15b is provided with an inner part 15b' and an outer part 15b'' separated by interconnecting webs 16b forming therebetween one or more ducts. Between the housing 1b and the ring 15b there is provided supporting webs 17b forming therebetween one or more channels through which the second fluid may flow from the nipple 8b to the peripheral channel 14b, which is arranged between two flanges 18b and 19b. Between the end cap 4b and the end wall 3b there is preferably a layer of glue 20b. A second layer of glue 21b is preferably arranged between the widened part 13b of the housing 1b and the end cap 4b. The attachment and the sealing may, however, also be provided as mentioned above by other means.

Furthermore, at filtration normally only one outlet is needed for the second fluid. Any inlet for the second fluid is not necessary at such a use.

## Claims

1. An apparatus for effecting mass and/or heat transfer, comprising:
a cylindrical housing (1) having first and second open ends interconnected by an inner surface;
a bundle of hollow fibres (2) arranged inside the housing and extending between first and second end walls (3) arranged adjacent the first and second open ends, respectively, of the housing;
a first end cap (4) having an engagement portion for cooperation with the housing for closing the first open end thereof, comprising first connector means (5) for passing a first fluid intended to flow through an interior of the bundle of hollow fibers (2), and second connector means (8) for passing a second fluid from an exterior of the bundle of hollow fibers (2);
a second end cap (4) for closing the second open end of the housing, comprising first connector means (6) for passing the first fluid;
**characterized** by a ring (15) arranged between the first end wall and the housing adjacent the first open end and the first end cap, for providing a fluid channel for the second fluid from the exterior of the bundle of hollow fibers and between the ring (15) and the inner wall surface to the second connector means (8).

2. An apparatus according to claim 1, **characterized** in that the second end cap is identical with the first end cap and that a second ring (15) is arranged between the second end wall and the housing adjacent the second open end and the second end cap, for providing a fluid channel for the second fluid at the exterior of the bundle of hollow fibers and between the ring (15) and the inner wall surface to the second connector means (8).

3. An apparatus according to claim 1 or 2, **characterized** in that the ring (15) is partly moulded into the corresponding end wall (3).

4. An apparatus according to any of the preceding claims, **characterized** in that the ring (15) consists of an inner portion (15b' in fig 3) abutting the bundle of hollow fibers (2), and an outer portion (15b'' in fig 3) abutting the housing (1) whereby the corresponding end wall may be moulded while the ring supports the bundle of hollow fibers.

5. An apparatus according to any of the preceding claims, **characterized** in that the inner wall surface of the housing (1) and an inner surface of the ring (15) are essentially in line, supporting the bundle of hollow fibers (2).

6. An apparatus according to claim 4, **characterized** in that the inner and outer portions (15b',15b'') are interconnected by several webs forming one or more ducts which are filled by the moulding material of the end wall.

7. An apparatus according to any of the preceding claims, **characterized** in that each end cap (4) is attached to the housing (1) and/or the end wall (3) by glueing.

8. An apparatus according to any of the preceding claims, **characterized** in that the housing (1) is provided at each end with a widened part (13) providing a peripheral channel (14) for the second fluid.

## Patentansprüche

1. Vorrichtung für Stoff- und/oder Wärmeübertragung mit
einem zylindrischen Gehäuse (1) mit ersten und zweiten offenen Enden, die durch eine Innenoberfläche miteinander verbunden sind,
einem Hohlfaserbündel (2), das im Inneren des Gehäuses angeordnet ist und sich zwischen ersten und zweiten Endwänden (3) erstreckt, die jeweils in Nachbarschaft zu den ersten und zweiten offenen Enden des Gehäuses angeordnet sind,
einer ersten Endkappe (4) mit einem Eingriffabschnitt zum Zusammenwirken mit dem Gehäuse für ein Verschließen des ersten offenen Endes desselben, mit einer ersten Verbindungseinrichtung (5) zum Führen eines ersten Fluids, das durch ein Inneres des Hohlfaserbündels (2) fließen soll, und einer zweiten Verbindungseinrichtung (8) zum Führen eines zweiten Fluids von einem Außenraum des Hohlfaserbündels (2) aus und
einer zweiten Endkappe (4) zum Verschließen des zweiten offenen Endes des Gehäuses mit einer ersten Verbindungseinrichtung (6) zum Führen des ersten Fluids,
**gekennzeichnet durch** einen Ring (15), der zwischen der ersten Endwand und dem Gehäuse in Nachbarschaft zu dem ersten offenen Ende und der ersten Endkappe angeordnet ist, um einen Fluidkanal für das zweite Fluid aus dem Außenraum des Hohlfaserbündels und zwischen dem Ring (15) und der Innenwandoberfläche zu der zweiten Verbindungseinrichtung (8) zu bekommen.

2. Vorrichtung nach Anspruch 1, **dadurch gekennzeichnet**, daß die zweite Endkappe mit der ersten Endkappe identisch ist und daß ein zweiter Ring (15) zwischen der zweiten Endwand und dem Gehäuse in Nachbarschaft zu dem zweiten offenen Ende und der zweiten Endkappe angeordnet ist, um einen Fluidkanal für das zweite Fluid am Außenraum des Hohlfaserbündels und zwischen dem Ring (15) und der Innenwandoberfläche zu der zweiten Verbindungseinrichtung (8) zu bekommen.

3. Vorrichtung nach Anspruch 1 oder 2, **dadurch gekennzeichnet**, daß der Ring (15) teilweise in die entsprechende Endwand (3) eingeformt ist.

4. Vorrichtung nach einem der vorausgehenden Ansprüche, **dadurch gekennzeichnet**, daß der Ring (15) aus einem Innenabschnitt (15b' in Fig. 3), der an das Hohlfaserbündel (2) angrenzt, und einem Außenabschnitt (15b'' in Fig. 3), der an das Gehäuse (1) angrenzt, besteht, wodurch die entsprechende Endwand geformt werden kann, während der Ring das Hohfaserbündel unterstützt.

5. Vorrichtung nach einem der vorausgehenden Ansprüche, **dadurch gekennzeichnet**, daß die Innenwandoberfläche des Gehäuses (1) und eine Innenoberfläche des Ringes (15) im wesentlichen in Linie ausgerichtet sind und das Hohlfaserbündel (2) unterstützen.

6. Vorrichtung nach Anspruch 4, **dadurch gekennzeichnet**, daß die Innen- und Außenabschnitte (15b', 15b'') durch mehrere Stoffbahnen miteinander verbunden sind welche eine oder mehrere Leitungen bilden, die durch das Formmaterial der Endwand gefüllt sind.

7. Vorrichtung nach einem der vorausgehenden Ansprüche, **dadurch gekennzeichnet**, daß jede Endkappe (4) an dem Gehäuse (1) und/oder der Endwand (3) durch Verleimen befestigt ist.

8. Vorrichtung nach einem der vorausgehenden Ansprüche, **dadurch gekennzeichnet**, daß das Gehäuse (1) an jedem Ende mit einem verbreiterten Teil (13) versehen ist, welches einen peripheren Kanal (14) für das zweite Fluid liefert.

## Revendications

1. Appareil pour effectuer un transfert de masse et/ou de chaleur, comprenant :
un corps cylindrique (1) ayant une première et une deuxième extrémités ouvertes interconnectées par une surface intérieure ;
un faisceau de fibres creuses (2) disposées à l'intérieur du corps et s'étendant entre une première et une deuxième parois d'extrémité (3) adjacentes aux première et deuxième extrémités ouvertes du corps, respectivement ;
un premier fond (4) ayant une partie d'accouplement pour coopération avec le corps de façon à fermer sa première extrémité ouverte, comportant un premier raccord (5) pour passage d'un premier fluide qui doit circuler à l'intérieur du faisceau de fibres creuses (2), et un deuxième raccord (8) pour passage d'un deuxième fluide venant d'un espace extérieur du faisceau de fibres creuses (2) ;
un deuxième fond (4) pour fermer la deuxième extrémité ouverte du corps, comportant un premier raccord (6) pour le passage du premier fluide ;
caractérisé par un anneau (15) agencé entre la première paroi d'extrémité et le corps, près de la première extrémité ouverte et du premier fond, pour définir un canal de fluide pour le deuxième fluide venant de l'extérieur du faisceau de fibres creuses et passant entre l'anneau (15) et la surface de paroi intérieure, vers le deuxième raccord (8).

2. Appareil suivant la revendication 1, caractérisé en ce que le deuxième fond est identique au premier fond, et en ce qu'un deuxième anneau (15) est disposé entre la deuxième paroi d'extrémité et le corps près de la deuxième extrémité ouverte et du deuxième fond, pour définir un canal de fluide pour le deuxième fluide à l'extérieur du faisceau de fibres creuses et entre l'anneau (15) et la surface de paroi intérieure, vers le deuxième raccord (8).

3. Appareil suivant la revendication 1 ou 2, caractérisé en ce que l'anneau (15) est partiellement moulé dans la paroi d'extrémité correspondante (3).

4. Appareil suivant une quelconque des revendications précédentes, caractérisé en ce que l'anneau (15) est constitué d'une partie intérieure (15b' sur la figure 3) en butée contre le faisceau de fibres creuses (2), et d'une partie extérieure (15b'' sur la figure 3) en butée contre le corps (1), de sorte que la paroi d'extrémité correspondante peut être moulée pendant que l'anneau supporte le faisceau de fibres creuses.

5. Appareil suivant une quelconque des revendications précédentes, caractérisé en ce que la surface de paroi intérieure du corps (1) et une surface intérieure de l'anneau (15) sont sensiblement en ligne, supportant le faisceau de fibres creuses (2).

6. Appareil suivant la revendication 4, caractérisé en ce que les parties intérieure et extérieure (15b', 15b'') sont interconnectées par plusieurs goussets définissant un ou plusieurs conduits qui sont remplis par la matière de moulage de la paroi d'extrémité.

7. Appareil suivant une quelconque des revendications précédentes, caractérisé en ce que chaque fond (4) est fixé au corps (1) et/ou à la paroi d'extrémité (3) par collage.

8. Appareil suivant une quelconque des revendications précédentes, caractérisé en ce que le corps (1) comporte, à chaque extrémité, une partie élargie (13) définissant un canal périphérique (14) pour le deuxième fluide.
